# EUROPÄISCHE PATENTSCHRIFT

(11) **EP 2 091 408 B1**
(45) Veröffentlichungstag und Bekanntmachung des Hinweises auf die Patenterteilung: **26.04.2017**
(21) Anmeldenummer: 07846563.0
(22) Anmeldetag: 13.11.2007
(51) Int. Cl.: A61B 3/00, A61B 3/107, A61B 3/15, A61B 3/10, A61B 3/11, A61B 3/117

(54) **OPHTHALMOLOGISCHES UNTERSUCHUNGSGERÄT**
OPHTHALMOLOGICAL EXAMINATION DEVICE
APPAREIL D'EXAMEN OPHTALMOLOGIQUE

(30) Priorität: 17.11.2006 DE 102006054774
(43) Veröffentlichungstag der Anmeldung: 26.08.2009
(73) Patentinhaber: Carl Zeiss Meditec AG, 07745 Jena (DE)
(72) Erfinder: BARTH, Roland, 07743 Jena (DE); BEHRENDT, Frank, 07743 Jena (DE); BERGNER, Roland, 07745 Jena (DE); VOIGT, Klaus-Ditmar, 07745 Jena (DE)
(74) Vertreter: Beck, Bernard
(86) Internationale Anmeldenummer: PCT/EP2007/009793
(87) Internationale Veröffentlichungsnummer: WO 2008/058699

(56) Entgegenhaltungen:
- WO-A-98/23202
- DE-A1- 3 541 726
- DE-A1- 19 715 212
- US-A- 3 718 386
- US-A- 4 861 155
- US-A- 5 735 283

## Beschreibung

Die Erfindung betrifft ein ophthalmologisches Untersuchungsgerät, bei dem Messungen des Auges vorgenommen und dokumentiert werden.

Bei der Untersuchung der Augen eines Patienten werden häufig auch Messungen vorgenommen, deren Ergebnisse dokumentiert werden und die daher auch reproduzierbar sein müssen.

Dazu zählen unter anderen die Hornhautkrümmung, die Vorderkammertiefe, Iris-Durchmesser (z.B. nach der Weiss-zu-Weiss-Methode) und die Länge der Augenachse. Damit diese Messungen reproduzierbar sind kommt es neben der Messgenauigkeit insbesondere auch darauf an, dass das Messgerät während der Messung exakt und wiederholbar zum Patientenauge positioniert ist.

Aus der US 5,463,430 ist ein Keratometer bekannt, bei dem zur Ermittlung der richtigen Messentfernung zwei verschiedene Markensysteme verwendet werden. Zunächst wird eine kreisförmige Hilfsmarkierung in den Strahlengang eingespiegelt, deren scheinbarer Durchmesser dem durchschnittlichen Durchmesser der Cornea eines Patienten entspricht. Mit dieser Markierung wird durch den Bediener die Ausrichtung als auch grob die richtige Entfernung des Auges des Patienten zum Messgerät bestimmt. Eine exaktere Bestimmung der Entfernung wird anschließend dadurch vorgenommen, dass Teile des zur eigentlichen Messung vorgesehenen Indexmarkensystems sowohl aus "endlich" als auch aus "unendlich" auf die Cornea projiziert werden. Zum Wechsel zwischen "endlich" und "unendlich" wird eine Kollimatorlinse zeitweise aus dem Projektionsstrahlengang ausgeschwenkt, welche zur eigentlichen Messung wieder eingeschwenkt werden muss. Bei Einstellung der korrekten Entfernung wird die Messung automatisch ausgelöst, was dem Bediener durch einen Farbwechsel der kreisförmigen Hilfsmarkierung angezeigt wird.

In der US 5,905,562 wird vorgeschlagen, die axiale Ausrichtung des Auges durch Projektion einer zusätzlichen Messmarke auf die Cornea, Aufnahme des sich ergebenden Bildes und Auswertung mittels Bildverarbeitung zu bestimmen, die Bestimmung der Messentfernung geschieht in gleicher Art und Weise wie in US 5,463,430. Auch hier wird nach korrekter Einstellung die Messung automatisch ausgelöst.

Die US 3 718 386 offenbart ein Perimeter, welches nach Auswertung der Patienteneingabe ein akustisches Signal für diesen abgibt.

Auch in der WO 1998/23202 wird unter bestimmten Umständen ein akustisches oder optisches Signal für den Patienten erzeugt.

Aus der US 5 735 283 ist ein Keratometer bekannt, bei welchem eine nicht näher bestimmte numerische Streuung als Qualitätsparameter herangezogen wird.

Diese Lösungen weisen eine Reihe von Nachteilen auf. So ist die Bestimmung der Messentfernung durch das notwendige Einschwenken der Kollektorlinse zeitaufwendig und kann daher bei Bewegung des Patienten zu falschen Messergebnissen führen, außerdem ist zusätzlicher mechanischer Aufwand notwendig. Es hat sich gezeigt, dass auch die automatische Auslösung der Messung bei den Lösungen nach dem Stand der Technik nicht immer optimale Ergebnisse ergibt, da z.B. die Abbildung des Auges durch in den Strahlengang des Messgerätes ragende Wimpern, ungenügend ausgebildeten Tränenfilm o.ä. verfälscht werden kann, was dann zu Fehlmessungen führen kann. Diese Zustände können von den Lösungen des Standes der Technik nicht erkannt werden, die automatische Auslösung kann daher auch stattfinden, wenn ungeeignete Messbedingungen vorliegen, was in der Folge zu falschen Messungen führen kann, welche aber prinzipbedingt nur schwer oder gar nicht als solche erkannt werden können.

Der Erfindung liegt die Aufgabe zu Grunde die Nachteile des Standes der Technik zu überwinden und ein ophthalmologisches Messgerät anzugeben, welches auch unter ungünstigen Bedingungen genaue Messergebnisse liefert.

Diese Aufgabe wird erfindungsgemäß durch ein ophthalmologisches Messgerät gelöst, bei welchem die Messsysteme mit einer Auswerteeinheit verbunden sind, welche die Einhaltung von Qualitätsparametern bzgl. der Messungen überprüft und ein entsprechendes Signal generiert.

Das erfindungsgemäße Verfahren zum Betrieb des ophthalmologischen Messgerätes zeichnet sich dadurch aus, dass die Messsysteme mehrere Messungen hintereinander durchführen, wobei für jede Messung Qualitätsparameter bestimmt werden und diese Qualitätsparameter von einer Auswerteeinheit überprüft werden. Wenn diese Überprüfung ergibt, dass eine ordnungsgemäße Messung möglich ist, wird ein Signal generiert, welches dem Benutzer anzeigt, dass jetzt die eigentliche Messung ausgelöst werden kann. Dieses Signal kann vorteilhaft ein optisches Signal sein, z.B. ein Farbwechsel einer Anzeige oder eine zusätzliche Anzeige. Es ist aber auch möglich, dass dieses Signal akustisch oder auch taktil erfolgt.

Der Benutzer hat damit die Möglichkeit, nachdem die Auswertung der Qualitätsparameter der Messungen ergeben hat, dass eine ordnungsgemäße Messung möglich sein sollte, aus seiner Erfahrung auch andere, bei der automatischen Bestimmung der Qualitätsparameter nicht berücksichtigte oder nicht berücksichtbare Zustände in seine Entscheidung, die endgültige Messung auszulösen, einfließen zu lassen.

Die Erfindung wird im Folgenden an Hand eines bevorzugten Ausführungsbeispiels, eines ophthalmologischen Messgerätes zur Messung der Hornhautkrümmung und der Vorderkammertiefe, näher erläutert. Das Messprinzip und der Aufbau eines solchen Gerätes sind beispielsweise in der Patentanmeldung DE 19 857 001, auf deren kompletten Inhalt hiermit Bezug genommen wird, ausführlich dargestellt.

Es zeigen
Fig. 1: den Messmodus Keratometer (Hornhautkrümmungsmessung)
Fig. 2: den Messmodus Vorderkammertiefe

### Keratometer:

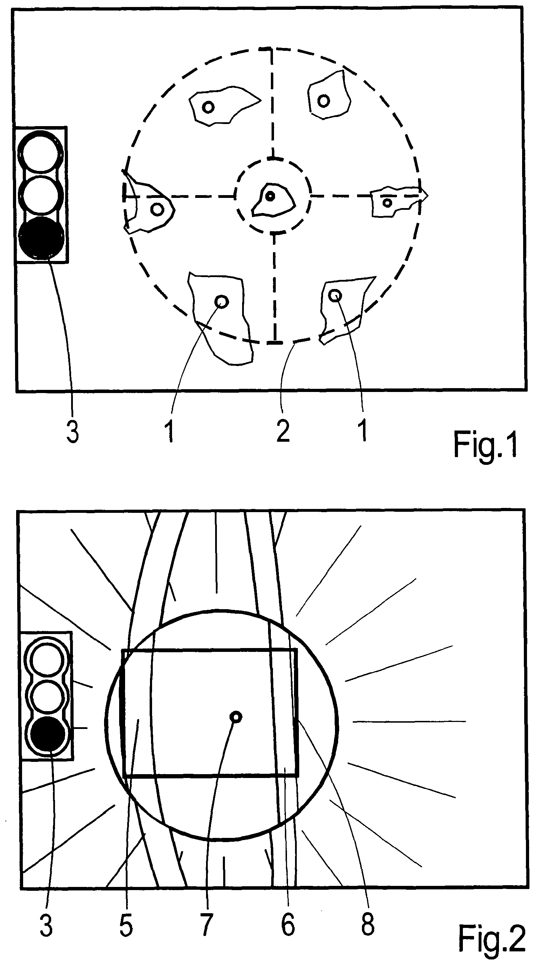

Fig. 1 zeigt den Messmodus so wie der dem Bediener auf seinem Bedienpult grafisch dargestellt wird.

Die Beleuchtung des Auges erfolgt mittels sechs IR-Dioden. Das von der Hornhaut reflektierte Licht wird auf eine CCD-Kamera abgebildet und aus dem Abstand der punktförmigen Reflexbilder 1 werden die Hornhautradien berechnet.

Diese sechs Messmarken 1 werden für eine interne automatische Bestimmung der Messqualität verwendet.

Zur Bestimmung der Messentfernung z wird die Größe der Abbildungen der einzelnen Messmarken als Funktion des Hornhautradius' ermittelt. Dazu werden während des Einstellvorgangs mehrere Messungen (z.B. 10 mal pro Sekunde) durchgeführt und jeweils die Bilder der sechs Reflexbilder 1 bewertet. Zu dieser Bewertung werden beispielsweise Radius, Fläche und Umfang der Reflexbilder rechentechnisch bestimmt (ggf. nach einer vorherigen Schwellwertbestimmung) und nach vorgegebenen Kriterien untersucht.

Dabei wird die Fokussierung, d.h. der Abstand für die schärfste Abbildung durch Suche nach dem Minimum der Flächen der Reflexbilder 1 gefunden.

Zur Bestimmung der x, y - Ablage wird ein hier kreisförmig dargestelltes Messfenster 2 auf dem Bild, welches die CCD-Kamera liefert, definiert, in welchem alle sechs Reflexbilder liegen müssen.

Um auszuschließen, dass ungenügender Tränenfilm, herabhängende Lider oder Wimpern die Messmarken beeinflussen, wird eine Formerkennung für die sechs Reflexbilder 1 durchgeführt. Dabei werden z.B. Rundheit, Umfang und Energie/Helligkeit ermittelt und ein Vergleich dieser Eigenschaften der sechs Bilder untereinander durchgeführt.

In Abhängigkeit der Erfüllung von definierten Kriterien wird beispielsweise ein Signal in Form einer Ampel 3, welche auf dem Bedienerdisplay dargestellt wird, von rot über gelb auf grün geschaltet. Rot bedeutet dabei, dass keine ordnungsgemäße Messung durchgeführt werden kann, in diesem Fall kann auch die Auslösung der Messung unterbunden werden; bei grün ist eine ordentliche Messung möglich und der Benutzer kann sie auslösen.

Kriterien für die Schaltung der Ampel auf "gelb" sind z.B. wenn die Standardabweichung der Flächen der einzelnen Reflexbilder 1 kleiner als 25% ist oder die Standardabweichung der Umfänge kleiner 20%. Für "grün" müssen die entsprechenden Standardabweichungen kleiner 15 bzw. 10% sein.

Es ist auch möglich bei "grün" die automatische Auslösung von mindestens einer Messung vorzunehmen wenn der Benutzer auf die eigene Bewertung verzichtet.

Alternativ ist es möglich ständig eine Messung des Hornhautradius durchzuführen und diese zusammen mit den Qualitätskriterien abzuspeichern um dann später die beste Messung anhand der Qualitätskriterien auszuwählen. Auch diese Auswahl kann wiederum automatisch durch rechentechnische Bewertung der Qualitätskriterien erfolgen.

### Vorderkammertiefe

Fig. 2 zeigt ebenfalls wie der Messmodus auf dem Bediendisplay dargestellt wird.

Das Auge wird seitlich durch einen Lichtspalt beleuchtet. Die dabei entstehenden Lichtschnitte am Auge (Hornhaut 5, Linse 6) werden auf eine CCD-Kamera abgebildet. Der Patient blickt zentral auf eine LED, der dabei entstehende Lichtreflex 7 wird ebenfalls auf die CCD-Kamera abgebildet.

Diese Lichtschnitte 5, 6 und der Reflexpunkt 7 werden für die automatische Bestimmung der Messqualität verwendet.

Zur Bestimmung der x, y - Ablage wird ein Messfenster 8 in dem Bild, welches die CCD-Kamera liefert definiert, in welchem die Lichtschnitte 5, 6 und das Bild des Fixierpunktes 7 liegen müssen.

Zur Bestimmung der Messentfernung z wird die Größe des Bildes des einzelnen Reflexpunktes 7 als Funktion des Hornhautradius' ermittelt. Dazu werden während des Einstellvorgangs mehrere Messungen (z.B. 10 mal pro Sekunde) durchgeführt und die nachfolgenden Untersuchungen durchgeführt:
Zur richtigen Einstellung des Gerätes zum Patientenauge wird die Lage und Größe des Bildes des einzelnen Punktes 7 (Bild des Fixierpunktes) zu den Lichtschnitten 5, 6 überwacht, dieser muss zwischen 5 Hornhaut- und 6 Linsenschnittbild liegen) und es wird detektiert, dass das Hornhautschnittbild 5 frei ist von Zusatzreflexen. Für die Größe des Bildes des Reflexpunktes 7 lässt sich aus dem vorher bestimmten Hornhautradius (siehe Keratometer-Messung) ein Erwartungswert bestimmen, dessen Einhaltung ebenfalls als Kriterium dienen kann.

In Abhängigkeit von der Einhaltung dieser Kriterien wird wieder eine Ampel 3, welche auf dem Bedienerdisplay dargestellt wird, von rot über gelb auf grün geschaltet und so dem Benutzer signalisiert, dass er die Messung auslösen kann.

Die Erfindung ist nicht an die dargestellten Ausführungsbeispiele gebunden, insbesondere können auch für andere Messverfahren entsprechende Kriterien aufgestellt und überwacht werden, ohne den Schutzbereich der Patentansprüche zu verlassen.

## Patentansprüche

1. Ophthalmologisches Messgerät zur Bestimmung von Hornhautradius oder Vorderkammertiefe bei welchem Messsysteme zur Bestimmung dieser Größen vorgesehen sind,
**gekennzeichnet dadurch, dass** die Messsysteme mit einer Auswerteeinheit verbunden sind, welche die Einhaltung von Qualitätsparametern bzgl. der Messungen überprüft und ein entsprechendes Signal für den Benutzer generiert und dass als Qualitätsparameter für die Bestimmung von Hornhautradius die Rundheit und/oder der Umfang der Reflexbilder von Messmarken eines Keratometers ermittelt wird oder dass als Qualitätskriterium für die Bestimmung der Vorderkammertiefe die Lage und Größe des Bildes des Fixierpunktes zu der Lage der Hornhaut und der Linse in Lichtschnittbildern des Auges ermittelt wird.

2. Ophthalmologisches Messgerät nach Anspruch 1, **gekennzeichnet dadurch, dass** das Signal von dem Benutzer optisch, akustisch oder taktil wahrnehmbar ist.

3. Verfahren zum Betrieb des ophthalmologischen Messgerätes nach Anspruch 1,
**gekennzeichnet dadurch,**
**dass** die Messsysteme mehrere Messungen hintereinander durchführen,
wobei für jede Messung Qualitätsparameter bestimmt werden,
diese Qualitätsparameter von einer Auswerteeinheit überprüft werden und das Ergebnis dieser Überprüfung dem Benutzer signalisiert wird.

4. Verfahren nach Anspruch 3, **gekennzeichnet dadurch, dass** die Messergebnisse und die erreichten Qualitätsparameter der einzelnen Messungen gespeichert werden und so einer späteren Auswertung zur Verfügung stehen.

5. Verfahren nach Anspruch 3, **gekennzeichnet dadurch, dass** dem Benutzer bei positiver Signalisierung die Entscheidung zur Auslösung der Messung überlassen wird.

6. Verfahren nach Anspruch 3, **gekennzeichnet dadurch, dass** bei positiver Signalisierung die Messung automatisch ausgelöst wird.

7. Verfahren nach Anspruch 3, **gekennzeichnet dadurch, dass** die Auslösung der Messung nicht möglich ist solange keine positive Signalisierung vorliegt.

## Claims

1. Ophthalmological measuring device for determining corneal radius or anterior chamber depth, in which measurement systems for determining these variables are provided,
**characterized in that** the measurement systems are connected to an evaluation unit which monitors the observance of quality parameters in respect of the measurements and generates a corresponding signal for the user and **in that** the roundness and/or the circumference of the reflection images of measurement marks of a keratometer is/are ascertained as quality parameter for determining corneal radius or **in that** the position and size of the image of the fixing point in relation to the position of the cornea and position of the lens in light section images of the eye are ascertained as quality criterion for determining the anterior chamber depth.

2. Ophthalmological measuring device according to Claim 1, **characterized in that** the signal is perceivable optically, acoustically or in tactile fashion by the user.

3. Method for operating the ophthalmological measuring device according to Claim 1, **characterized in that** the measurement systems perform a plurality of measurements in succession,
wherein quality parameters are determined for each measurement,
these quality parameters are monitored by an evaluation unit
and the result of this monitoring is signalled to the user.

4. Method according to Claim 3, **characterized in that** the measurement results and the obtained quality parameters of the individual measurements are stored and are thus available for a later evaluation.

5. Method according to Claim 3, **characterized in that** the decision for triggering the measurement is left to the user's discretion in the case of a positive signal.

6. Method according to Claim 3, **characterized in that** the measurement is triggered automatically in the case of a positive signal.

7. Method according to Claim 3, **characterized in that** the measurement cannot be triggered for as long as no positive signal is present.

## Revendications

1. Appareil de mesure ophtalmologique destiné à déterminer le rayon de courbure de la cornée ou la profondeur de la chambre antérieure, dans lequel il est prévu des systèmes de mesure destinés à déterminer lesdites grandeurs,
**caractérisé en ce que** les systèmes de mesure sont connectés à une unité d'analyse qui vérifie le respect de paramètres de qualité ou des mesures et génère un signal correspondant pour l'utilisateur et **en ce que** la rotondité et/ou la circonférence de l'image de réflexion de repères de mesure d'un kératomètre en tant que paramètres de qualité pour la détermination du rayon de courbure de la cornée, ou **en ce que** la position et la taille de l'image du point de fixation par rapport à la position de la cornée et du cristallin sont déterminées dans des images de coupe optique de l'oeil en tant que critère de qualité pour la détermination de la profondeur de la chambre antérieure.

2. Appareil de mesure ophtalmologique selon la revendication 1, **caractérisé en ce que** le signal est perceptible par l'utilisateur de manière optique, acoustique ou tactile.

3. Procédé de mise en fonctionnement de l'appareil ophtalmologique selon la revendication 1, **caractérisé en ce que** les systèmes de mesure effectuent plusieurs mesures consécutives, dans lequel, pour chaque mesure, des paramètres de qualité sont déterminés, **en ce que** lesdits paramètres de qualité sont vérifiés par une unité d'analyse et **en ce que** le résultat de ladite vérification est signalé à l'utilisateur.

4. Procédé selon la revendication 3, **caractérisé en ce que** les résultats de mesure et les paramètres de qualité obtenus des mesures individuelles sont stockés et sont ainsi mis à disposition pour une analyse ultérieure.

5. Procédé selon la revendication 3, **caractérisé en ce que**, lors d'une signalisation positive, la décision du déclenchement de la mesure revient à l'utilisateur.

6. Procédé selon la revendication 3, **caractérisé en ce que**, lors d'une signalisation positive, la mesure est déclenchée automatiquement.

7. Procédé selon la revendication 3, **caractérisé en ce que** le déclenchement de la mesure n'est pas possible tant qu'aucune signalisation positive n'est présente.
